# EUROPEAN PATENT SPECIFICATION

(11) **EP 3 300 081 B1**
(45) Date of publication and mention of the grant of the patent: **23.06.2021**
(21) Application number: 17192235.4
(22) Date of filing: 20.09.2017
(51) Int. Cl.: G16H 15/00

(54) **METHOD AND DEVICE FOR SECURING MEDICAL RECORD**
VERFAHREN UND VORRICHTUNG ZUR SICHERUNG DER KRANKENAKTE
PROCÉDÉ ET DISPOSITIF DE SÉCURISATION DE DOSSIERS MÉDICAUX

(30) Priority: 22.09.2016 US 201615272861
(43) Date of publication of application: 28.03.2018
(73) Proprietor: Siemens Healthcare GmbH, 91052 Erlangen (DE)
(72) Inventor: Elachithaya Rajagopal, Laxmikantha, 560040 Bangalore (IN); Rangapura Shettappa, Chandrashekara, 560100 Bengaluru (IN)
(74) Representative: Patentanwälte Bals & Vogel

(56) References cited:
- EP-A1- 2 194 477
- US-A1- 2010 063 930
- US-A1- 2010 082 371
- US-A1- 2015 379 198

## Description

The present disclosure relates to the field of securing health information, and more particularly to the field of securing medical record associated with a patient.

Privacy of patient information in a medical record is a fundamental right of every patient and safeguarding such information is of utmost importance. Masking of patient data is a form of information sanitization performed on medical records wherein sensitive patient information is either replaced, encrypted or removed in order to maintain anonymity of the patient. Masking of patient information in a medical record enables sharing of patient data over various platforms without concerns of loss of privacy. Therefore, masking of patient information ensures that the patient remains unidentifiable by the recipient of the information.

As the patient data becomes digital, the need for such data to be stored in the cloud environment also increases. The security risk of the patient information that is stored in the cloud increases, as it is in the public domain. If the medical record of the patient is contained in a Digital Imaging and Communications in Medicine (DICOM) file, the patient data is masked according to a default configuration which is required as per the standard requirements of data privacy. The protected health information (PHI) tags present in the DICOM file are identified and are masked according to the default standard privacy configuration. Such masking of patient information in a medical record is common for all studies uploaded from a healthcare service provider to cloud environment.

However, some of the private tags present in the DICOM file may not be masked and therefore may reveal patient information. The standard masking procedure of a DICOM file also does not take into consideration the user specific data privacy configuration that may be defined by a user or patient in order to protect his privacy on the medical record. Moreover, there also exists a need to inform the user or patient about any violation of data privacy that may occur when their medical record on the cloud environment is shared with an external recipient.

Reference US 2010/063930 discloses A method, system and device for secure mobile healthcare Selections, which a user can, from a mobile device, authorize the comparison of pangenetic (genetic and epigenetic) data with data profiles corresponding to healthcare products, services and service providers to determine which are the most appropriate for a particular consumer.

Reference US 2015/0379198 discloses Various embodiments, which manage access to patient health-related information in a health care patient portal, wherein In one embodiment, a determination is made that a user has accessed the health care patient portal and a set of electronic health-related records associated with the user is identified. Further, a source identifier associated with each record is also identified. The source identifier is associated with an entity that provided the electronic health-related record to the health care patient portal. A sharing status associated with each record is determined. The sharing status indicates that the associated electronic healthrelated record is one of sharable and non-sharable with an entity other than the user. The records are presented to the user in the health care patient portal. A least one record is presented with the source identifier and the sharing status associated with the record. Reference US 2010/0082371 discloses a system and method for managing health care information. A data network system includes a document parser, upon request, parses a patient's record into a plurality of categorized biographical and/or clinical data entries, where each of the categorized biographical and/or clinical data entries may be associated with a code identifier.

Therefore, the object of the invention is to provide a method and a system to secure medical record associated with a patient according to the privacy configuration defined by the patient.

The invention comprises a computer-implemented method, a system and a non-transitory computer-readable storage medium as defined by the appended claims.

A computer-implemented method and system of securing medical record associated with a patient is disclosed. In one aspect, a method includes obtaining the medical record associated with the patient from a patient database, wherein the medical record comprises a patient identifier, patient information and medical data. The method also includes determining preferences of the patient with respect to privacy of the patient information in the medical record. Furthermore, the method comprises masking one or more data fields in the patient information based on the determined preferences. Additionally, the method comprises generating the medical record containing the masked data fields in the patient information.

The method further discloses, that, wherein determining the preferences of the patient with respect to the privacy of the patient information comprises:
a. determining whether any preferences are specified by the patient with respect to the privacy of the patient information in a patient profile information;
b. if any preferences are specified, determining one or more data fields in the patient information to be masked based on the preferences specified by the patient; and
c. if no preferences are specified, requesting the patient to provide the preferences with respect to the privacy of the patient information.

The method can further comprise:
a. determining whether any of the masked data fields are tampered, wherein the masked data fields are tampered by unmasking the masked data fields; and
b. if any of the masked data fields are tampered, generating an alert indicating the tampering of the masked data fields to the patient.
Masking the data fields in the patient information can comprise:
a. determining the data fields in the patient information masked according to preferences set by a healthcare service provider;
b. comparing the masked data fields with the one or more data fields specified in the preferences of the patient;
c. determining the data fields which are to be masked and the data fields which are not to be masked according to the preferences of the patient;
d. masking the data fields which are determined to be masked according to the preferences of the patient; and
e. unmasking the masked data fields which are not determined to be masked according to the preferences of the patient.

Preferred is a method, wherein masking the data fields of the patient information comprises:
a. determining a privacy level associated with each of the data fields of the patient information based on the preferences with respect to the privacy of the patient; and
b. masking the one or more data fields in the patient information according to the privacy level.

The method can further comprise setting preferences of the patient with respect to privacy of the patient information as default preferences for masking the patient information.

Further, the method can comprise:
a. receiving a request from a server to share the masked patient information with a specified entity; and
b. sharing the masked patient information with the specified entity, if such a request is received from the server.

In another aspect, a system for securing a medical record associated with a patient includes a processing unit; a patient database coupled to the processing unit and a memory coupled to the processing unit. The memory comprises a masking module configured for obtaining a medical record associated with a patient from a patient database. Furthermore, the masking module is configured for determining preferences of the patient with respect to privacy of the patient information in the medical record. Additionally, the masking module is also configured for masking one or more data fields in the patient information based on the determined preferences. Moreover, the masking module is also configured for generating the medical record containing the masked data fields in the patient information.

A system is preferred, wherein in determining the preferences of the patient with respect to privacy of the patient information, the masking module is configured to:
a. determine whether any preferences are specified by the patient with respect to the privacy of the patient information based on the patient identifier;
b. if any preferences are specified, determine one or more data fields in the patient information to be masked based on the preferences specified by the patient; and
c. if no preferences are specified, request the patient to provide the preferences with respect to the privacy of the patient information.

Further, the system can comprise a tamper proof module configured to:
a. determine whether any of the masked data fields are tampered, wherein the masked data fields are tampered by unmasking the masked data fields; and
b. if any of the masked data fields are tampered, generate an alert indicating the tampering of the masked data fields to the patient.

Preferred is a system, wherein in masking the data fields, the masking module is configured to:
a. determine a privacy level associated with each of the data fields of the patient information based on the preferences with respect to the privacy of the patient; and
b. mask the one or more data fields in the patient information according to a privacy level.

Preferred is a system, wherein in masking the data fields in the patient information, the masking module (103) is configured to:
a. determine whether any of the data fields in the patient information are masked according to the preferences set by a healthcare service provider;
b. if any of the data fields are masked, compare the masked data fields with the one or more data fields specified in the preferences of the patient;
c. determine the data fields which are to be masked and the data fields which are not to be masked according to the preferences of the patient;
d. mask the data fields which are determined to be masked according to the preferences of the patient; and
e. unmask the masked data fields which are not determined to be masked according to the preferences of the patient.

Preferred is a system, further comprising a preference module configured to set preferences of the patient with respect to privacy of the patient information as default preferences for masking the patient information.

In yet another aspect, a non-transitory computer-readable storage medium having machine readable instructions stored therein, that when executed by the server, causes the server to perform the method steps as described above. In particular a non-transitory computer-readable storage medium is provided having machine-readable instructions stored therein, that when executed by a server, cause the server to perform the method steps comprising:
a. obtaining a medical record associated with a patient from a medical record database, wherein the medical record comprises a patient identifier, patient health information and medical data;
b. determining preferences of the patient with respect to privacy of the patient information in the medical record;
c. masking one or more data fields in the patient information based on the determined preferences; and
d. generating the medical record containing the masked data fields in the patient information.

Further, a storage medium is preferred, wherein in determining the preferences of the patient with respect to privacy of the patient information, the instructions cause the server to perform the method steps comprising:
a. determining whether any preferences are specified by the patient with respect to the privacy of the patient information based on the patient identifier;
b. if any preferences are specified, determining one or more data fields in the patient information to be masked based on the preferences specified by the patient; and
c. if no preferences are specified, requesting the patient to provide the preferences with respect to the privacy of the patient information.

A storage medium is preferred, wherein the instructions cause the server to perform the method steps comprising:
a. determining whether any of the masked data fields are tampered; and
b. if any of the masked data fields are tampered, generating an alert indicating the tampering of the mask data fields to the patient.

Preferred is a storage medium, wherein the instructions cause the server (101) to perform the method steps comprising:
a. determining whether any of the data fields in the patient information are masked according to preferences set by a healthcare service provider;
b. if any of the data fields are masked, comparing the masked data fields with the one or more data fields specified in the preferences of the patient;
c. determining the data fields which are to be masked and the data fields which are not to be masked according to the preferences of the patient;
d. masking the data fields which are determined to be masked according to the preferences of the patient; and
e. unmasking the masked data fields which are not determined to be masked according to the preferences of the patient.

A storage medium is preferred, wherein in masking the data fields in the patient information, the instructions cause the processor (202) to perform the method steps comprising:
a. determining a privacy level associated with each of the data fields of the patient information based on the preferences with respect to the privacy of the patient; and
b. masking the one or more data fields in the patient information according to a privacy level.

This summary is provided to introduce a selection of concepts in a simplified form that are further described below in the following description. It is not intended to identify features or essential features of the claimed subject matter. Furthermore, the claimed subject matter is not limited to implementations that solve any or all disadvantages noted in any part of this disclosure.

The present invention is further described hereinafter with reference to illustrated embodiments shown in the accompanying drawings, in which:
- FIG 1: illustrates a block diagram of a client-server architecture which provides geometric modelling of components representing different parts of a real world object, according to an embodiment.
- FIG 2: illustrates a block diagram of a data processing system in which an embodiment for securing a medical record associated with a patient can be implemented.
- FIG 3: illustrates a flowchart of an exemplary method of securing a medical record associated with a patient, according to one embodiment.
- FIG 4: illustrates a flowchart of an exemplary method of generating masked data fields in the patient information according to the preferences specified by the patient, according to an embodiment.
- FIG 5: illustrates a flowchart of an exemplary method of masking data fields in the patient information according to the preferences specified by the patient, according to an embodiment.
- FIG 6: illustrates a flowchart of an exemplary method of generating an alert indicating tampering of masked data fields, according to an embodiment.
- FIG 7A: illustrates a flowchart of an exemplary method of masking the data fields in the patient information according to the privacy levels associated with the data fields, according to an embodiment.
- FIG 7B: illustrates an example of privacy keys that may be associated with the data fields in the patient information.
- FIG 8: illustrates a flowchart of an exemplary method of setting a default privacy preference for the patient information, according to an embodiment.
- FIG 9A: illustrates an embodiment of a graphical user interface for the patient information.
- FIG 9B: illustrates an embodiment of a graphical user interface displaying the data fields in the patient information in a medical record.
- FIG 9C: illustrates an embodiment of a graphical user interface displaying the medical record of the patient after the data fields in the patient information have been masked.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details. In other instances, well known materials or methods have not been described in detail in order to avoid unnecessarily obscuring embodiments of the present disclosure. While the disclosure is susceptible to various modifications and alternative forms, specific embodiments thereof are shown by way of example in the drawings and will herein be described in detail. It should be understood, however, that there is no intent to limit the disclosure to the particular forms disclosed, but on the contrary, the disclosure is to cover all modifications, equivalents, and alternatives falling within the scope of the present disclosure.

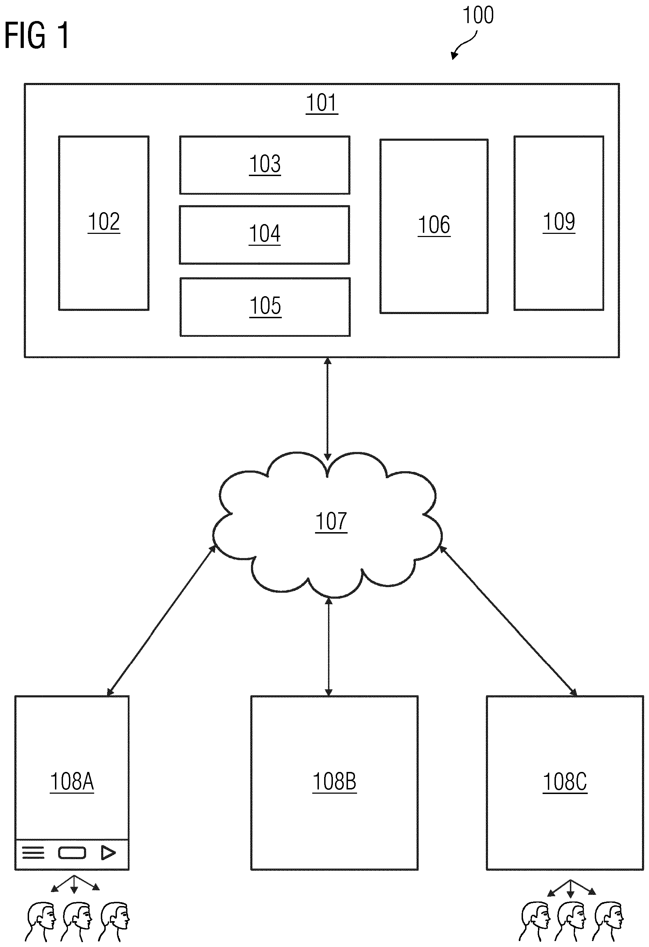

FIG 1 provides an illustration of a block diagram of a client-server architecture that is a geometric modelling of components representing different parts of real-world objects, according to an embodiment. The client-server architecture 100 includes a server 101 and a plurality of client devices 108A-C. Each of the client devices 108A-C are connected to the server 101 via a network 107, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 107, for example, the internet. The cloud computing environment provides on-demand network access to a shared pool of the configurable computing physical and logical resources. The server 101 may include a medical record database 102 that comprises medical records of patients that are to be secured. The server 101 may include a masking module 103 that performs the masking of one or more data fields of patient information contained in the medical record of the patient. The server 101 may also include a preference module 104 that determines privacy preferences defined by the patient. The server 101 may also include patient profile information 106 that stores the privacy preferences defined by the patient. The server 101 may include a tamper proof module 105 that generates an alert when the data fields that are masked according to the privacy preferences defined by the patient are tampered with. The masked data fields may be tampered by unmasking the masked data fields. Additionally, the server 101 may include a network interface 109 for communicating with the client devices 108A-C via the network 107.

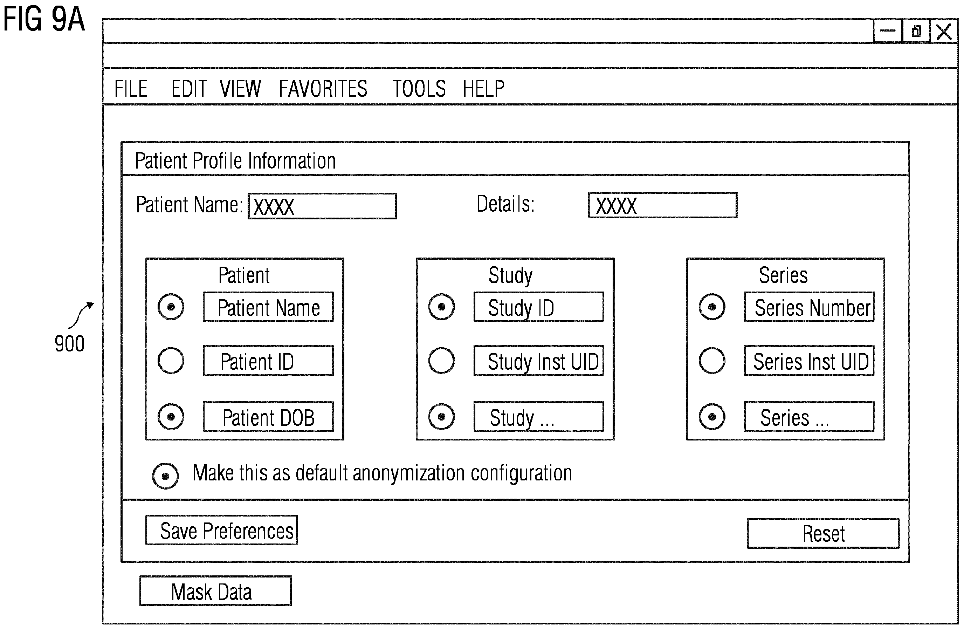

The client devices 108A-C includes a user device 108A, used by a user. In an embodiment, the user device 108A may be used by a patient to define the privacy preferences for masking one or more data fields in the patient information, on the patient profile information 106. The patient profile information 106 on the server 101 can be accessed by the user via a graphical user interface of an end user web application. An embodiment of the graphical user interface 900 for the patient profile information has been illustrated in FIG 9A. In an embodiment, the user can define the privacy preference on the patient profile information and save the privacy preference on the server 101. The privacy preference defined by the user is saved in an XML format and may be applied as default for masking the one or more data fields in the patient information. In another embodiment, the client may be a healthcare service provider system 108B. The healthcare service provider system 108B interacts with the server 101 via the network interface 109 to upload medical records of the patient on the server 101. The healthcare service provider system 108B may also be used to access the masked patient information via a graphical user interface. The healthcare service provider system 108B may send a request to the server 101 to share the masked patient information with other specified entities via the network 107. In another embodiment, the client device is a device used by a practitioner 108C to access patient profile information 106 on the server 101 via the network 107. The practitioner device 108C may send a request to the server 101 to share the masked patient information with other specified entities via the network 107.

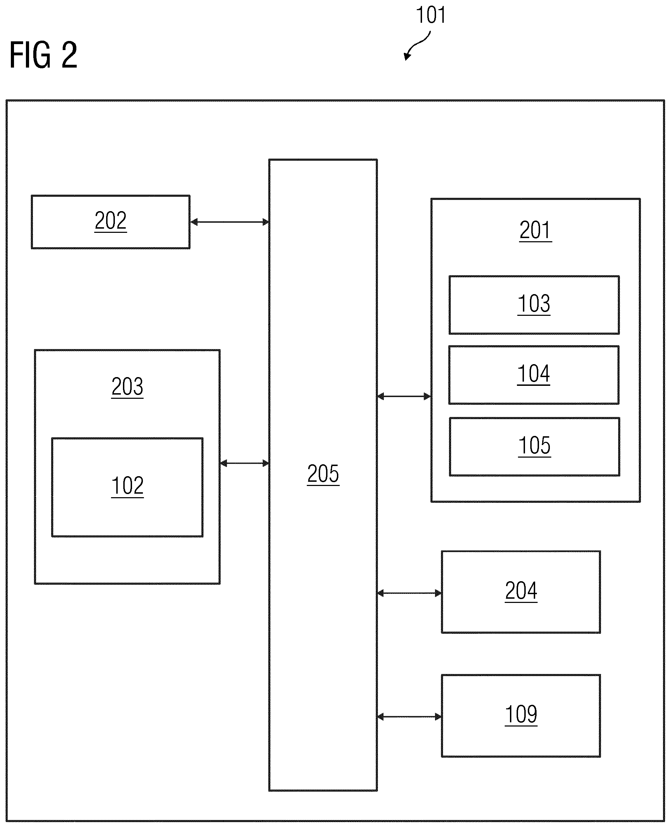

FIG 2 is a block diagram of a data processing system in which an embodiment can be implemented, for example, as a system to secure medical record associated with a patient, configured to perform the processes as described therein. It is appreciated that the server 101 is an exemplary implementation of the data processing system in FIG 1. In FIG 1, the data processing system 101 comprises a memory 201, a processor 202, a storage unit 203, an input unit 204, a network interface 109 and a bus 205.

The processor 202, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processor 202 may also include embedded controllers, such as generic or programmable logic devices or arrays, application specific integrated circuits, single-chip computers, and the like.

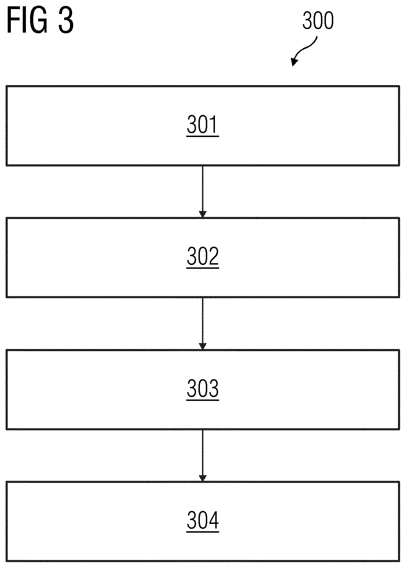

The memory 201 may be volatile memory and non-volatile memory. The memory 201 may be coupled for communication with the processor 202. The processor 202 may execute instructions and/or code stored in the memory 201. A variety of computer-readable storage media may be stored in and accessed from the memory 201. The memory 201 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 includes a masking module 103, a preference module 104 and a tamper proof module 105 stored in the form of machine-readable instructions on any of the above-mentioned storage media and may be in communication to and executed by processor 202. When executed by the processor 202, the masking module 103 causes the processor 202 to perform masking of one or more data fields of the patient information according to the privacy preferences defined by the patient. When executed by the processor 202, the preference module 104 causes the processor 202 to to set preferences of the patient with respect to privacy of the patient information as default preferences for masking the patient information. When executed by the processor 202, the tamper proof module 105 causes the processor 202 to generate alert for the patient if any of the data fields of the patient information are tampered with. Method steps executed by the processor 202 to achieve the abovementioned functionality are elaborated upon in detail in FIG 3, 4, 5, 6, 7A and 8.

The storage unit 203 may be a non-transitory storage medium which stores a medical record database 102. The medical record database 102 is a repository of medical information related to one or more patients that is maintained by a healthcare service provider. The input unit 204 may include input means such as keypad, touch-sensitive display, camera (such as a camera receiving gesture-based inputs), etc. capable of receiving input signal such as a medical record including patient information to be masked. The bus 205 acts as interconnect between the processor 202, the memory 201, the storage unit 203, the communication interface 109 and the input unit 204.

Those of ordinary skilled in the art will appreciate that the hardware depicted in FIG 2 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. The depicted example is provided for the purpose of explanation only and is not meant to imply architectural limitations with respect to the present disclosure.

A data processing system in accordance with an embodiment of the present disclosure includes an operating system employing a graphical user interface. The operating system permits multiple display windows to be presented in the graphical user interface simultaneously with each display window providing an interface to a different application or to a different instance of the same application. A cursor in the graphical user interface may be manipulated by a user through the pointing device. The position of the cursor may be changed and/or an event such as clicking a mouse button, generated to actuate a desired response.

One of various commercial operating systems, such as a version of Microsoft Windows™, a product of Microsoft Corporation located in Redmond, Washington may be employed if suitably modified. The operating system is modified or created in accordance with the present disclosure as described.

Disclosed embodiments provide systems and methods for securing medical record associated with a patient. In particular, the systems and methods may perform masking of one or more data fields in the patient information according to privacy preferences defined by the patient.

FIG 3 illustrates a flowchart of an exemplary method 300 of securing medical record associated with a patient. At step 301, a medical record associated with a patient is obtained from the medical record database 102. As used herein, "medical record" may refer to data specific to health of a patient and may include patient identifier, patient information, and medical data. The medical record database may be stored on the server 101. At step 302, the preferences of the patient with respect to the privacy of the patient information in the medical record are determined. The preferences for privacy of the patient information may be stored in the patient profile information 106 on the server 101. In an alternate embodiment, the patient profile information 106 may also be stored in a cloud computing environment. The patient profile information 106 may include information such as patient demographics, medical history, etc. The patient profile information 106 may also include privacy preferences with respect to the patient information in the medical record. The patient may be allowed to configure the preferences with respect to the privacy of the patient information contained in a medical record. The defined preferences with respect to privacy of the patient information are stored in the patient profile information 106. At step 303, one or more data fields in the patient information are masked based on the determined preferences with respect to privacy of the patient information. As used herein, "masking" refers to hiding or anonymizing one or more data fields related to patient information such that confidential information related to the patient is not disclosed to another entity. Masking may include removing, modifying, or replacing information which are contained in the one or more data fields of the patient information. At step 304, the medical record containing masked data fields in the patient information is generated.

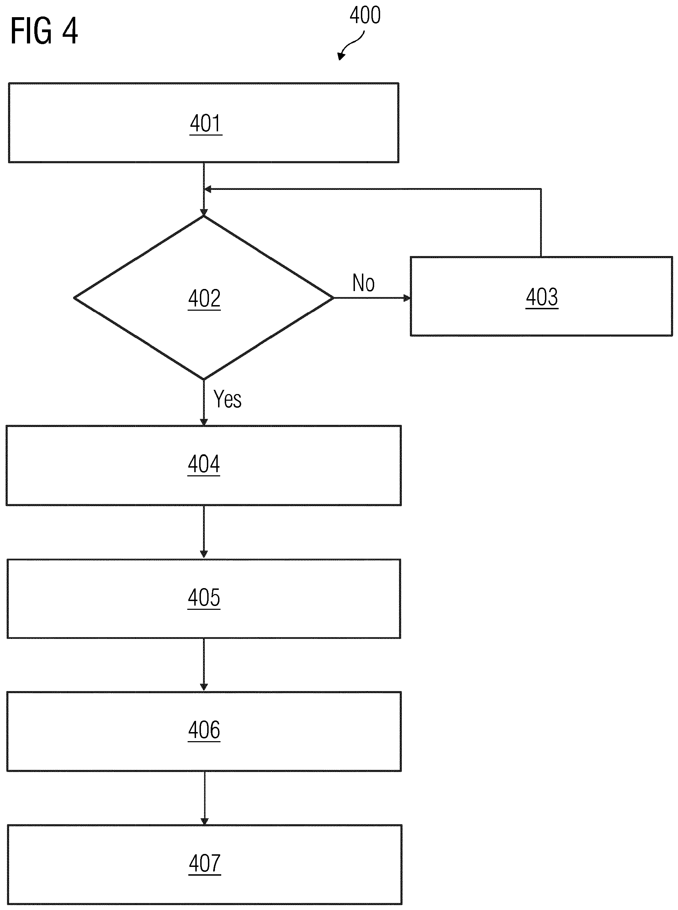

FIG 4 illustrates a flowchart of an exemplary method 400 of generating the medical record containing masked data fields in the patient information according to the preferences defined by the patient with respect to privacy. At step 402, once the medical record is obtained from the medical record database 102, the patient profile information 106 is accessed to determine if any privacy preferences are specified by the patient. In an embodiment, the preferences with respect to privacy of patient information contained in the medical record may be specified by the patient in the patient profile information 106. If any preferences with respect to privacy of patient information is specified by the patient in the patient profile information 106, the one or more data fields in the patient information is masked based on the specified preferences, at step 404. In an embodiment, if no preferences with respect to privacy of the patient information are specified by the patient, at step 403, a request to provide a privacy preference is made to the patient. The patient profile information 106 may be accessed by the patient via a graphical user interface. The graphical user interface for the patient profile information is illustrated in FIG 9A. The graphical user interface may be used by the patient to specify the preferences in the patient profile information. The graphical user interface for the patient profile information 106 may include information related to one or more data fields that may be chosen by the patient to be masked. The patient may select one or more data fields in the patient information to be masked, for example, by clicking a mouse button on the option. In one embodiment, the click of the mouse button may proceed in checking or unchecking the data fields of the patient information on the graphical user interface. If the preferences are provided by the patient, at step 404, the one or more data fields in the patient information are masked based on the determined preferences. At step 405, the medical record containing the masked data fields in the patient information is generated based on the privacy preferences specified by the patient. The generated medical record containing the masked data fields is saved to the server 101 at step 406. The masked medical record may be accessed by the patient or a healthcare service provider from the server 101. At step 407, the medical record containing masked patient information may be shared with a specified entity. In an embodiment, a request for sharing the medical record may be received by the server 101 through the network interface 109 from a specified entity. If a request for sharing the masked medical record is received, the masked medical record is shared with the specified entity. In an alternate embodiment, the medical record with masked data fields may be saved in the cloud computing environment.

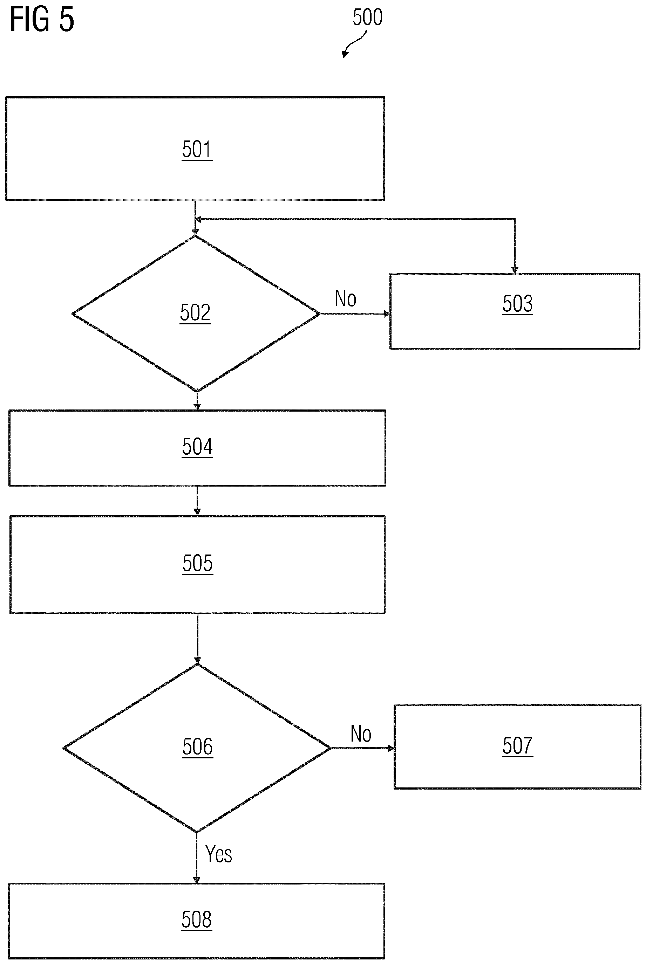

FIG 5 illustrates a flowchart of an exemplary method 500 of masking data fields in the patient information in the medical record according to the preferences specified by the patient, according to an embodiment. At step 501, the data fields in the patient information included in the medical record that have been masked by the healthcare service provider are determined. In an embodiment, the healthcare service provider may mask the data fields in the patient information according to the standard requirement of data privacy for a medical record. Therefore, the data fields in the patient information may be modified or removed according to the standard privacy preferences associated with a medical record. The healthcare service provider may mask the data fields manually or the masking may be performed as an automated batch process, wherein masking is performed for one or more medical records simultaneously. At step 502, the patient profile information 106 is accessed to determine if any preferences with respect to privacy of patient information are specified by the patient. If no preferences are specified by the patient in the patient profile information 106, the patient is requested to specify the preferences, at step 503. In an embodiment, the request generated may be indicated in the graphical user interface as a notification which may occur on the display unit of the user device 108A used by the patient. At step 504, when the privacy preferences with respect to the data fields in the patient information have been specified, the masked data fields are compared with one or more data fields specified by the patient. The privacy preference specified by the healthcare service provider for each of the data fields is compared to determine if the privacy preference of the data fields matches with the privacy preference specified by the patient. At step 505, the data fields that are to be masked and the data fields that are not to be masked are determined according to the preferences specified by the patient. Privacy preference of a data field in the patient information determines a privacy configuration for that data field. The privacy configuration may vary according to the strictness of the privacy that is associated with the content of the data field in the patient information. At step 506, whether the data fields are masked according to the preferences of the patient is determined. On comparison, if privacy configuration of one or more data fields in the patient information does not match with the privacy configuration defined according to the preferences specified by the patient, at step 507, the data fields that have not been masked by the healthcare service providers are masked according to the patient preferences and the data fields which are not to be masked according to the patient preferences but have been masked by healthcare providers are unmasked. The medical record with masked data fields in the patient information according to the privacy preferences of the patient is generated at step 508 and may be saved on the server 101. In another embodiment, the generated medical record with masked data fields may also be stored in the cloud computing environment.

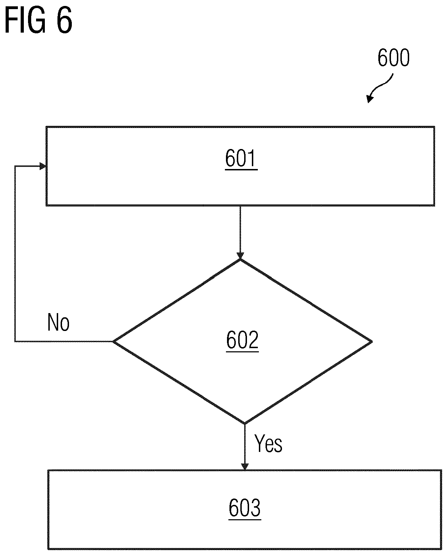

FIG 6 provides an illustration of a flowchart of an exemplary method 600 of generating an alert indicating tampering of masked data fields, according to an embodiment. The medical record containing the masked data fields in the patient information may be saved on the server 101 or in a cloud computing environment. At step 602, the saved medical record is accessed to determine if any of the masked data fields have been tampered. The masked data fields are tampered by unmasking the masked data fields. The medical record with masked data fields may be accessed from the server 101 via a network interface 109, for example, by a healthcare service provider. The healthcare service provider may unmask the data fields in the patient information which were masked according to the patient preferences with respect to privacy of patient information. The healthcare service provider may also mask certain data fields which, according to the privacy preferences of the patient, have not been masked. An alert for such masking/unmasking of data fields in the patient information, which may not be in conformation with the privacy preferences specified by the patient, may be generated for the patient at step 603. The alert is received on the user device 108A via the network interface 109 and may be displayed on the display unit of the user device 108A used by the patient as a notification. The notification may occur as a pop-up window on the display unit of the user device 108A and may contain information about the tampering of data fields in the patient information. The user device 108A may also be configured to generate a sound alert when the notification is received on the user device 108A via the network interface 109.

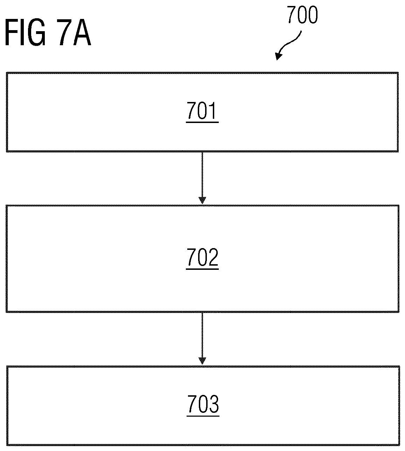

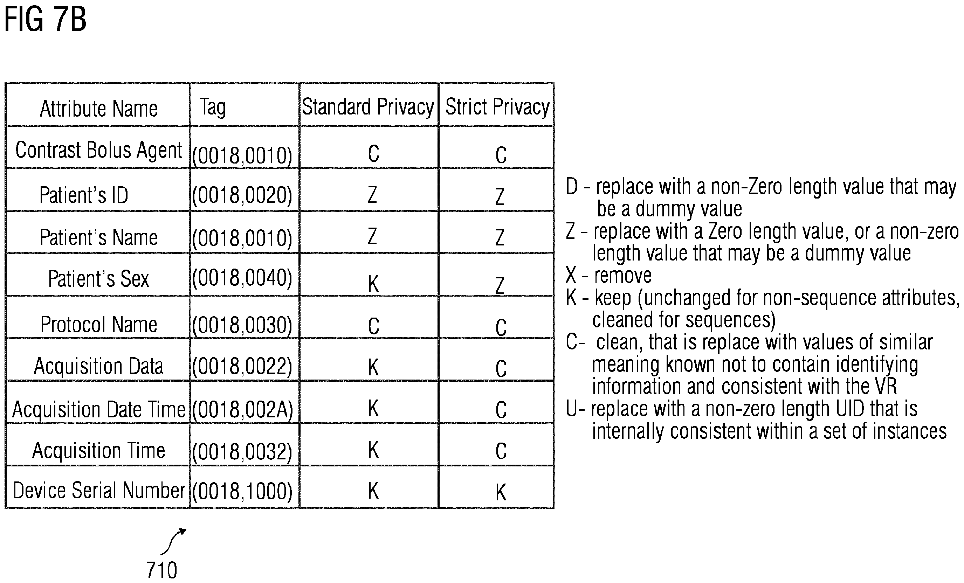

FIG 7A illustrates a flowchart of an exemplary method 700 of masking the data fields in the patient information according to the privacy levels associated with the data fields, according to an embodiment. Masking of the data fields in the patient information is performed according to the privacy preferences specified by the patient in the patient profile information 106. At step 701, a privacy level associated with each of the data fields in the patient information is determined based on the privacy preferences specified by the patient. As used herein, "privacy level" refers to the strictness of the privacy that may be associated with the data fields in the patient information. A privacy key is associated with each of the data fields, which determines the privacy level of the data fields in the patient information. Based on the strictness of the privacy, the privacy key associated with a data field may change. FIG 7B provides a table 710 illustrating an example of the privacy keys that may be associated with the data fields in the patient information. The standard privacy parameters define the standard privacy configuration for data fields associated with patient information. In an embodiment, the healthcare service providers may refer to the standard privacy parameters to mask the data fields in the patient information. Each of the privacy keys defines how masking of the content of the data fields is performed. For example, privacy key 'D' replaces the contents of the data fields with a non-zero length value that may be a dummy value. Therefore, based on the privacy key associated with the data fields, the contents of the data fields may be replaced, removed or kept unchanged at step 702.

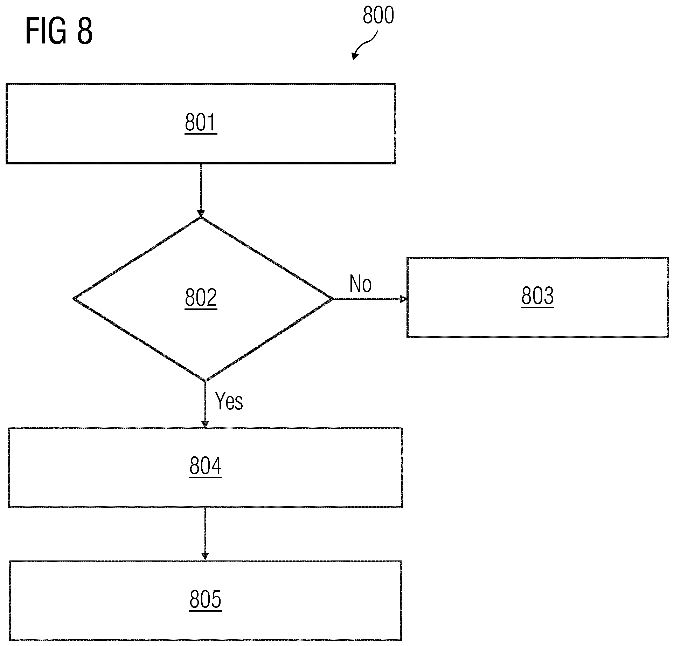

FIG 8 illustrates a flowchart of an exemplary method 800 of setting a default privacy preference for the patient information, according to an embodiment. The data fields in the patient information are masked according to the privacy preferences specified by the patient in the patient profile information 106. If the privacy preferences are not specified by the patient, at step 803, a request is made to the patient to specify the privacy preferences with respect to the patient information. Once the privacy preferences are specified by the patient, the privacy preferences may be saved in the patient profile information 106 on the server 101. The graphical user interface for the patient profile information may contain an option to save the privacy preferences specified by the patient in the patient profile information. In an embodiment, the privacy preferences specified by the patient are saved if the patient selects the option to save the specified privacy preferences. FIG 9A illustrates an example of the graphical user interface 900 for the patient profile information. The graphical user interface 900 as illustrated in FIG 9A includes one or more options for one or more data fields that may be chosen to be masked by the patient. In an embodiment, the graphical user interface 900 also includes one or more radio buttons against the options for data fields, which may be clicked using a mouse button to choose the masking preference. In the embodiment, the graphical user interface 900 includes an option to save the privacy preferences specified by the patient for the data fields in the patient information. In the embodiment, the option to save may be included in the graphical user interface in the form of a button that can be clicked on by a mouse so as to trigger an action for saving the preferences in the patient profile information. If the privacy preferences specified by the patient are saved, the preferences are applied as default for masking all the upcoming medical records associated with the patient, at step 805. The graphical user interface 900 provides a radio button that can be chosen by the patient if he/she intends to use the specified privacy preferences as default preference for all the subsequent medical records associated with him/her. The patient may click on the designated radio button with a mouse click so as to trigger an action for application of privacy preferences as default preference. The patient may access the patient profile information 106 to modify the privacy preferences. The modified privacy preferences may be applicable only for the subsequent masking of medical records associated with the patient.

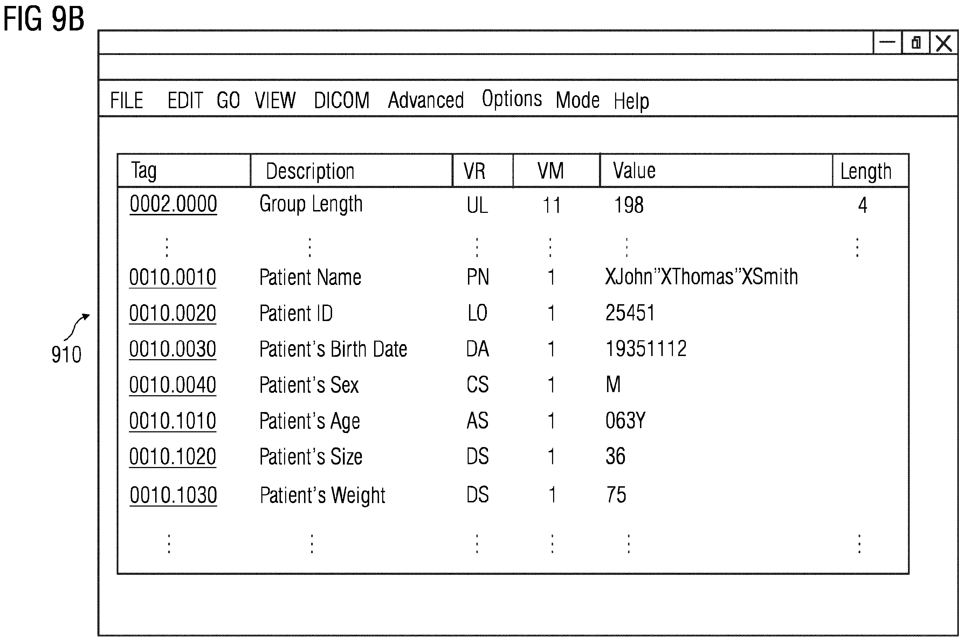

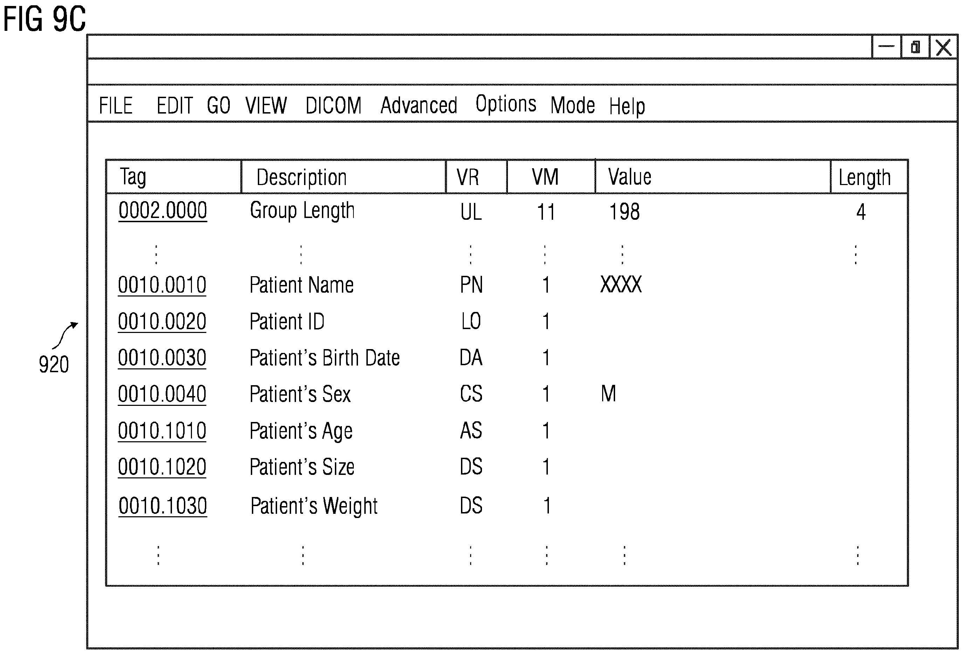

FIG 9B illustrates an embodiment of the graphical user interface 910 displaying the data fields in the patient information in a medical record, according to an embodiment. Each of the data fields in the patient information is associated with a tag for identification of the type of information. In the graphical user interface 910, the values associated with the data fields are not masked and therefore represent the actual values as may be present in a medical record of a patient. FIG 9C illustrates a graphical user interface 920 displaying the medical record of the patient after the data fields in the patient information have been masked, according to an embodiment. In the graphical user interface 920, one or more data fields in the patient information are masked according to the privacy preferences specified by the patient in the patient profile information 106. For example, the name of the patient has been replaced with a non-zero value and the date of birth of the patient has been hidden or removed.

## Claims

1. A computer-implemented method (300) of securing a medical record associated with a patient, the method comprising:
a. obtaining the medical record associated with the patient from a medical record database, wherein the medical record comprises a patient identifier, patient information and medical data;
b. determining preferences of the patient with respect to privacy of the patient information in the medical record;
c. masking one or more data fields in the patient information based on the determined preferences; and
d. generating the medical record containing the masked data fields in the patient information,
wherein masking the data fields of the patient information comprises:
determining a privacy level associated with each of the data fields of the patient information based on the preferences with respect to the privacy of the patient; and
masking the one or more data fields in the patient information according to the privacy level,
wherein masking refers to hiding or anonymizing data fields of the medical record by removing, modifying or deleting information contained in the data fields related to patient information,
wherein determining the preferences of the patient with respect to the privacy of the patient information comprises:
a. determining whether any preferences are specified by the patient with respect to the privacy of the patient information in a patient profile information;
b. if any preferences are specified, determining one or more data fields in the patient information to be masked based on the preferences specified by the patient; and **characterized in that**
c. if no preferences are specified, requesting the patient to provide the preferences with respect to the privacy of the patient information.

2. The method (300) according to claim 1, further comprising:
a. determining whether any of the masked data fields are tampered, wherein the masked data fields are tampered by unmasking the masked data fields; and
b. if any of the masked data fields are tampered, generating an alert indicating the tampering of the masked data fields to the patient.

3. The method (300) according to claim 1, wherein masking the data fields in the patient information comprises:
a. determining the data fields in the patient information masked according to preferences set by a healthcare service provider;
b. comparing the masked data fields with the one or more data fields specified in the preferences of the patient;
c. determining the data fields which are to be masked and the data fields which are not to be masked according to the preferences of the patient;
d. masking the data fields which are determined to be masked according to the preferences of the patient; and
e. unmasking the masked data fields which are not determined to be masked according to the preferences of the patient.

4. The method (300) according to any of the preceding claims, further comprising setting preferences of the patient with respect to privacy of the patient information as default preferences for masking the patient information.

5. The method (300) according to any of the preceding claims, further comprising:
a. receiving a request from a server to share the masked patient information with a specified entity; and
b. sharing the masked patient information with the specified entity, if such a request is received from the server.

6. A system (100) comprising:
a. a processing unit (202);
b. a patient database coupled to the processing unit (202);
c. a memory (201) coupled to the processing unit (202), the memory (201) comprising a masking module (103) configured for:
- obtaining a medical record associated with a patient from a medical record database (102), wherein the medical record comprises a patient identifier, patient health information and medical data;
- determining preferences of the patient with respect to privacy of the patient information in the medical record;
- masking one or more data fields in the patient information based on the determined preferences; and
- generating the medical record containing the masked data fields in the patient information,
wherein in masking the data fields, the masking module (103) is configured to:
determine a privacy level associated with each of the data fields of the patient information based on the preferences with respect to the privacy of the patient; and
mask the one or more data fields in the patient information according to a privacy level,
wherein masking refers to hiding or anonymizing data fields of the medical record by removing, modifying or deleting information contained in the data fields related to patient information,
wherein in determining the preferences of the patient with respect to privacy of the patient information, the masking module (103) is configured to:
a. determine whether any preferences are specified by the patient with respect to the privacy of the patient information based on the patient identifier;
b. if any preferences are specified, determine one or more data fields in the patient information to be masked based on the preferences specified by the patient; and **characterized in that**
c. if no preferences are specified, request the patient to provide the preferences with respect to the privacy of the patient information.

7. The system (100) according to claim 6, further comprising a tamper proof module (105) configured to:
a. determine whether any of the masked data fields are tampered, wherein the masked data fields are tampered by unmasking the masked data fields; and
b. if any of the masked data fields are tampered, generate an alert indicating the tampering of the masked data fields to the patient.

8. The system (100) according to claim 6, wherein in masking the data fields in the patient information, the masking module (103) is configured to:
a. determine whether any of the data fields in the patient information are masked according to the preferences set by a healthcare service provider;
b. if any of the data fields are masked, compare the masked data fields with the one or more data fields specified in the preferences of the patient;
c. determine the data fields which are to be masked and the data fields which are not to be masked according to the preferences of the patient;
d. mask the data fields which are determined to be masked according to the preferences of the patient; and
e. unmask the masked data fields which are not determined to be masked according to the preferences of the patient.

9. The system (100) according to claim 6, further comprising a preference module (104) configured to set preferences of the patient with respect to privacy of the patient information as default preferences for masking the patient information.

10. A non-transitory computer-readable storage medium having machine-readable instructions stored therein, that when executed by a server (101), cause the server (101) to perform the method steps comprising:
a. obtaining a medical record associated with a patient from a medical record database (102), wherein the medical record comprises a patient identifier, patient health information and medical data;
b. determining preferences of the patient with respect to privacy of the patient information in the medical record;
c. masking one or more data fields in the patient information based on the determined preferences; and
d. generating the medical record containing the masked data fields in the patient information,
wherein in masking the data fields in the patient information, the instructions cause the processor (202) to perform the method steps comprising:
determining a privacy level associated with each of the data fields of the patient information based on the preferences with respect to the privacy of the patient; and
masking the one or more data fields in the patient information according to a privacy level,
wherein masking refers to hiding or anonymizing data fields of the medical record by removing, modifying or deleting information contained in the data fields related to patient information, wherein in determining the preferences of the patient with respect to privacy of the patient information, the instructions cause the server (101) to perform the method steps comprising:
a. determining whether any preferences are specified by the patient with respect to the privacy of the patient information based on the patient identifier;
b. if any preferences are specified, determining one or more data fields in the patient information to be masked based on the preferences specified by the patient; and **characterized in that**
c. if no preferences are specified, requesting the patient to provide the preferences with respect to the privacy of the patient information.

11. The storage medium according to claim 10, wherein the instructions cause the server (101) to perform the method steps comprising:
a. determining whether any of the masked data fields are tampered; and
b. if any of the masked data fields are tampered, generating an alert indicating the tampering of the mask data fields to the patient.

12. The storage medium according to any of the preceding claims 10 to 11, wherein the instructions cause the server (101) to perform the method steps comprising:
a. determining whether any of the data fields in the patient information are masked according to preferences set by a healthcare service provider;
b. if any of the data fields are masked, comparing the masked data fields with the one or more data fields specified in the preferences of the patient;
c. determining the data fields which are to be masked and the data fields which are not to be masked according to the preferences of the patient;
d. masking the data fields which are determined to be masked according to the preferences of the patient; and
e. unmasking the masked data fields which are not determined to be masked according to the preferences of the patient.

## Patentansprüche

1. Computerimplementiertes Verfahren (300) zum Sichern einer Krankenakte, die einem Patienten zugeordnet ist, wobei das Verfahren umfasst:
a. Erhalten der dem Patienten zugeordneten Krankenakte aus einer Krankenaktendatenbank, wobei die Krankenakte einen Patientenidentifizierer, Patienteninformationen und medizinische Daten umfasst;
b. Bestimmen der Präferenzen des Patienten in Bezug auf die Privatsphäre der Patienteninformationen in der Krankenakte;
c. Maskieren eines oder mehrerer Datenfelder in den Patienteninformationen basierend auf den bestimmten Präferenzen; und
d. Erzeugen der Krankenakte, die die maskierten Datenfelder in den Patienteninformationen umfasst,
wobei das Maskieren der Datenfelder der Patienteninformationen umfasst:
Bestimmen eines Privatsphäreniveaus, das jedem der Datenfelder der Patienteninformationen zugeordnet ist, basierend auf den Präferenzen in Bezug auf die Privatsphäre des Patienten; und
Maskieren des einen oder der mehreren Datenfelder in den Patienteninformationen entsprechend des Privatsphäreniveaus,
wobei sich das Maskieren auf das Verbergen oder Anonymisieren von Datenfeldern der Krankenakte durch Entfernen, Ändern oder Löschen von Informationen in den Datenfeldern bezieht, die sich auf Patienteninformationen beziehen,
wobei das Bestimmen der Präferenzen des Patienten in Bezug auf die Privatsphäre der Patienteninformationen umfasst:
a. Bestimmen, ob vom Patienten irgendwelche Präferenzen in Bezug auf die Privatsphäre der Patienteninformationen in einer Patientenprofilinformation angegeben sind;
b. wenn irgendwelche Präferenzen angegeben sind, Bestimmen eines oder mehrerer Datenfelder in den Patienteninformationen, die maskiert werden sollen, basierend auf den vom Patienten angegebenen Präferenzen; und **dadurch gekennzeichnet, dass**
c. wenn keine Präferenzen angegeben sind, Auffordern des Patienten, die Präferenzen in Bezug auf die Privatsphäre der Patienteninformationen anzugeben.

2. Verfahren (300) nach Anspruch 1, ferner umfassend:
a. Bestimmen, ob irgendeines der maskierten Datenfelder manipuliert ist, wobei die maskierten Datenfelder durch Demaskieren der maskierten Datenfelder manipuliert sind; und
b. wenn eines der maskierten Datenfelder manipuliert ist, Erzeugen einer Warnmeldung, die dem Patienten die Manipulation der maskierten Datenfelder anzeigt.

3. Verfahren (300) nach Anspruch 1, wobei das Maskieren der Datenfelder in den Patienteninformationen umfasst:
a. Bestimmen der Datenfelder in den Patienteninformationen, die gemäß den von einem Anbieter von Gesundheitsdienstleistungen eingestellten Präferenzen maskiert sind;
b. Vergleichen der maskierten Datenfelder mit dem einen oder den mehreren Datenfeldern, die in den Präferenzen des Patienten angegeben sind;
c. Bestimmen der Datenfelder, die maskiert werden sollen, und der Datenfelder, die nicht maskiert werden sollen, entsprechend den Präferenzen des Patienten;
d. Maskieren der Datenfelder, die gemäß den Präferenzen des Patienten als maskiert bestimmt sind; und
e. Demaskieren der maskierten Datenfelder, die nach den Präferenzen des Patienten nicht als maskiert bestimmt sind.

4. Verfahren (300) nach einem der vorhergehenden Ansprüche, das ferner das Einstellen von Präferenzen des Patienten in Bezug auf die Privatsphäre der Patienteninformationen als Standardpräferenzen zum Maskieren der Patienteninformationen umfasst.

5. Verfahren (300) nach einem der vorhergehenden Ansprüche, ferner umfassend:
a. Empfangen einer Aufforderung von einem Server, die maskierten Patienteninformationen mit einer angegebenen Entität zu teilen; und
b. Weitergeben der maskierten Patienteninformationen an die angegebene Entität, wenn eine solche Aufforderung vom Server empfangen wird.

6. System (100), das Folgendes umfasst:
a. eine Verarbeitungseinheit (202);
b. eine Patientendatenbank, die mit der Verarbeitungseinheit (202) gekoppelt ist;
c. einen Speicher (201), der mit der Verarbeitungseinheit (202) gekoppelt ist, wobei der Speicher (201) ein Maskierungsmodul (103) umfasst, das konfiguriert ist zum:
- Erhalten einer dem Patienten zugeordneten Krankenakte aus einer Krankenaktendatenbank (102), wobei die Krankenakte einen Patientenidentifizierer, Patientengesundheitsinformationen und medizinische Daten umfasst;
- Bestimmen der Präferenzen des Patienten in Bezug auf die Privatsphäre der Patienteninformationen in der Krankenakte;
- Maskieren eines oder mehrerer Datenfelder in den Patienteninformationen basierend auf den bestimmten Präferenzen; und
- Erzeugen der Krankenakte, die die maskierten Datenfelder in den Patienteninformationen umfasst,
wobei zum Maskieren der Datenfelder das Maskierungsmodul (103) konfiguriert ist zum:
Bestimmen eines Privatsphäreniveaus, das jedem der Datenfelder der Patienteninformationen zugeordnet ist, basierend auf den Präferenzen in Bezug auf die Privatsphäre des Patienten; und
Maskieren des einen oder der mehreren Datenfelder in den Patienteninformationen entsprechend des Privatsphäreniveaus,
wobei sich das Maskieren auf das Verbergen oder Anonymisieren von Datenfeldern der Krankenakte durch Entfernen, Ändern oder Löschen von Informationen in den Datenfeldern bezieht, die sich auf Patienteninformationen beziehen,
wobei das Maskierungsmodul (103) bei der Bestimmung der Präferenzen des Patienten in Bezug auf die Privatsphäre der Patienteninformationen konfiguriert ist, zum:
a. Bestimmen, ob vom Patienten irgendwelche Präferenzen in Bezug auf die Privatsphäre der Patienteninformationen basierend auf dem Patientenidentifizierer angegeben sind;
b. wenn irgendwelche Präferenzen angegeben sind, Bestimmen eines oder mehrerer Datenfelder in den Patienteninformationen, die maskiert werden sollen, basierend auf den vom Patienten angegebenen Präferenzen; und **dadurch gekennzeichnet, dass**
c. wenn keine Präferenzen angegeben sind, Auffordern des Patienten, die Präferenzen in Bezug auf die Privatsphäre der Patienteninformationen vorzusehen.

7. System (100) nach Anspruch 6, das ferner ein manipulationssicheres Modul (105) umfasst, das konfiguriert ist, zum:
a. Bestimmen, ob irgendeines der maskierten Datenfelder manipuliert ist, wobei die maskierten Datenfelder durch Demaskieren der maskierten Datenfelder manipuliert sind; und
b. wenn eines der maskierten Datenfelder manipuliert ist, Erzeugen einer Warnmeldung, die dem Patienten die Manipulation der maskierten Datenfelder anzeigt.

8. System (100) nach Anspruch 6, wobei beim Maskieren der Datenfelder in den Patienteninformationen das Maskierungsmodul (103) konfiguriert ist, zum:
a. Bestimmen, ob irgendeines der Datenfelder in den Patienteninformationen gemäß den von einem Anbieter von Gesundheitsdienstleistungen eingestellten Präferenzen maskiert ist;
b. wenn irgendeines der Datenfelder maskiert ist, Vergleichen der maskierten Datenfelder mit dem einen oder den mehreren Datenfeldern, die in den Präferenzen des Patienten angegeben sind;
c. Bestimmen der Datenfelder, die maskiert werden sollen, und der Datenfelder, die nicht maskiert werden sollen, entsprechend den Präferenzen des Patienten;
d. Maskieren der Datenfelder, die gemäß den Präferenzen des Patienten als maskiert bestimmt sind; und
e. Demaskieren der maskierten Datenfelder, die nach den Präferenzen des Patienten nicht als maskiert bestimmt sind.

9. System (100) nach Anspruch 6, das ferner ein Präferenzmodul (104) umfasst, das so konfiguriert ist, dass es Präferenzen des Patienten in Bezug auf die Privatsphäre der Patienteninformationen als Standardpräferenzen zum Maskieren der Patienteninformationen einstellt.

10. Nicht-transitorisches computerlesbares Speichermedium mit darin gespeicherten maschinenlesbaren Anweisungen, die, wenn sie von einem Server (101) ausgeführt werden, den Server (101) veranlassen, die Verfahrensschritte durchzuführen, die Folgendes umfassen:
a. Erhalten einer dem Patienten zugeordneten Krankenakte aus einer Krankenaktendatenbank (102), wobei die Krankenakte einen Patientenidentifizierer, Patientengesundheitsinformationen und medizinische Daten umfasst;
b. Bestimmen der Präferenzen des Patienten in Bezug auf die Privatsphäre der Patienteninformationen in der Krankenakte;
c. Maskieren eines oder mehrerer Datenfelder in den Patienteninformationen basierend auf den bestimmten Präferenzen; und
d. Erzeugen der Krankenakte, die die maskierten Datenfelder in den Patienteninformationen umfasst,
wobei zum Maskieren der Datenfelder in den Patienteninformationen die Anweisungen den Prozessor (202) veranlassen, die Verfahrensschritte auszuführen, die umfassen:
Bestimmen eines Privatsphäreniveaus, das jedem der Datenfelder der Patienteninformationen zugeordnet ist, basierend auf den Präferenzen in Bezug auf die Privatsphäre des Patienten; und
Maskieren des einen oder der mehreren Datenfelder in den Patienteninformationen entsprechend des Privatsphäreniveaus,
wobei sich das Maskieren auf das Verbergen oder Anonymisieren von Datenfeldern der Krankenakte durch Entfernen, Ändern oder Löschen von Informationen in den Datenfeldern bezieht, die sich auf Patienteninformationen beziehen,
wobei bei der Bestimmung der Präferenzen des Patienten in Bezug auf die Privatsphäre der Patienteninformationen die Anweisungen den Server (101) veranlassen, die Verfahrensschritte durchzuführen, die Folgendes umfassen:
a. Bestimmen, ob vom Patienten irgendwelche Präferenzen in Bezug auf die Privatsphäre der Patienteninformationen basierend auf dem Patientenidentifizierer angegeben sind;
b. wenn irgendwelche Präferenzen angegeben sind, Bestimmen eines oder mehrerer Datenfelder in den Patienteninformationen, die maskiert werden sollen, basierend auf den vom Patienten angegebenen Präferenzen; und **dadurch gekennzeichnet, dass**
c. wenn keine Präferenzen angegeben sind, Auffordern des Patienten, die Präferenzen in Bezug auf die Privatsphäre der Patienteninformationen vorzusehen.

11. Speichermedium nach Anspruch 10, wobei die Anweisungen den Server (101) veranlassen, die Verfahrensschritte durchzuführen, die umfassen:
a. Bestimmen, ob irgendeines der maskierten Datenfelder manipuliert ist; und
b. wenn eines der maskierten Datenfelder manipuliert ist, Erzeugen einer Warnmeldung, die dem Patienten die Manipulation der maskierten Datenfelder anzeigt.

12. Speichermedium nach einem der vorhergehenden Ansprüche 10 bis 11, wobei die Anweisungen den Server (101) veranlassen, die Verfahrensschritte durchzuführen, die umfassen:
a. Bestimmen, ob irgendeines der Datenfelder in den Patienteninformationen gemäß den von einem Anbieter von Gesundheitsdienstleistungen eingestellten Präferenzen maskiert ist;
b. wenn irgendeines der Datenfelder maskiert ist, Vergleichen der maskierten Datenfelder mit dem einen oder den mehreren Datenfeldern, die in den Präferenzen des Patienten angegeben sind;
c. Bestimmen der Datenfelder, die maskiert werden sollen, und der Datenfelder, die nicht maskiert werden sollen, entsprechend den Präferenzen des Patienten;
d. Maskieren der Datenfelder, die gemäß den Präferenzen des Patienten als maskiert bestimmt sind; und
e. Demaskieren der maskierten Datenfelder, die nach den Präferenzen des Patienten nicht als maskiert bestimmt sind.

## Revendications

1. Procédé mis en oeuvre par ordinateur (300) de sécurisation d'un dossier médical associé à un patient, le procédé comprenant :
a. obtenir le dossier médical associé au patient à partir d'une base de données de dossiers médicaux, dans lequel le dossier médical comprend un identifiant de patient, des informations du patient et des données médicales ;
b. déterminer les préférences du patient en ce qui concerne la confidentialité des informations du patient dans le dossier médical ;
c. masquer un ou plusieurs champs de données dans les informations du patient sur la base des préférences déterminées ; et
d. générer le dossier médical contenant les champs de données masqués dans les informations du patient,
dans lequel le masquage des champs de données des informations du patient comprend :
déterminer un niveau de confidentialité associé à chacun des champs de données des informations du patient sur la base des préférences concernant la confidentialité du patient ; et
masquer un ou plusieurs champs de données dans les informations du patient sur la base de niveau de confidentialité,
où le masquage comprend cacher ou rendre anonymes des champs de données du dossier médical en supprimant, modifiant ou effaçant les informations contenues dans les champs de données relatifs aux informations du patient,
dans lequel la détermination des préférences du patient en ce qui concerne la confidentialité des informations du patient comprend :
a. déterminer si des préférences sont spécifiées par le patient en ce qui concerne la confidentialité des informations du patient dans les informations du profil du patient ;
b. si des préférences sont spécifiées, déterminer un ou plusieurs champs de données dans les informations du patient à masquer sur la base des préférences spécifiées par le patient ; et **caractérisé en ce que**
c. si aucune préférence n'est spécifiée, demander au patient de fournir les préférences en ce qui concerne la confidentialité des informations du patient.

2. Procédé (300) selon la revendication 1, comprenant en outre :
a. déterminer si l'un des champs de données masqués est altéré, dans lequel les champs de données masqués sont altérés en démasquant les champs de données masqués ; et
b. si l'un des champs de données masqués est altéré, générer une alerte indiquant au patient l'altération des champs de données masqués.

3. Procédé (300) selon la revendication 1, dans lequel le masquage des champs de données dans les informations du patient comprend :
a. déterminer les champs de données dans les informations du patient masqués selon les préférences définies par un fournisseur de services de soins de santé ;
b. comparer les champs de données masqués avec le ou les champs de données spécifiés dans les préférences du patient ;
c. déterminer les champs de données qui doivent être masqués et les champs de données qui ne doivent pas être masqués sur la base des préférences du patient ;
d. masquer les champs de données qui sont déterminés comme devant être masqués selon les préférences du patient ; et
e. démasquer les champs de données masqués qui ne sont pas déterminés comme devant être masqués selon les préférences du patient.

4. Procédé (300)' selon l'une des revendications précédentes, comprenant en outre le paramétrage des préférences du patient en ce qui concerne la confidentialité des informations du patient en tant que préférences par défaut pour masquer les informations du patient.

5. Procédé (300) selon l'une des revendications précédentes, comprenant en outre :
a. recevoir une demande d'un serveur pour partager les informations du patient masquées avec une entité spécifiée ; et
b. partager les informations du patient masquées avec l'entité spécifiée, si une telle demande est reçue du serveur.

6. Système (100) comprenant :
a. une unité de traitement (202) ;
b. une base de données de patient couplée à l'unité de traitement (202) ;
c. une mémoire (201) couplée à l'unité de traitement (202), la mémoire (201) comprenant un module de masquage (103) configuré pour :
- obtenir un dossier médical associé à un patient à partir d'une base de données de dossiers médicaux (102), dans lequel le dossier médical comprend un identifiant de patient, des informations sur la santé du patient et des données médicales ;
- déterminer les préférences du patient en ce qui concerne la confidentialité des informations du patient dans le dossier médical ;
- masquer un ou plusieurs champs de données dans les informations du patient sur la base des préférences déterminées ; et
- générer le dossier médical contenant les champs de données masqués dans les informations du patient,
dans lequel, lors du masquage des champs de données, le module de masquage (103) est configuré pour :
déterminer un niveau de confidentialité associé à chacun des champs de données des informations du patient sur la base des préférences concernant la confidentialité du patient ; et
masquer le ou les champs de données dans les informations du patient sur la base d'un niveau de confidentialité,
où le masquage comprend cacher ou rendre anonymes des champs de données du dossier médical en supprimant, modifiant ou effaçant les informations contenues dans les champs de données relatifs aux informations du patient,
dans lequel, en déterminant les préférences du patient en ce qui concerne la confidentialité des informations du patient, le module de masquage (103) est configuré pour :
a. déterminer si des préférences sont spécifiées par le patient en ce qui concerne la confidentialité des informations du patient sur la base de l'identifiant du patient ;
b. si des préférences sont spécifiées, déterminer un ou plusieurs champs de données dans les informations du patient à masquer sur la base des préférences spécifiées par le patient ; et **caractérisé en ce que**
c. si aucune préférence n'est spécifiée, demander au patient de fournir les préférences en ce qui concerne la confidentialité des informations du patient.

7. Système (100) selon la revendication 6, comprenant en outre un module d'inviolabilité (105) configuré pour :
a. déterminer si l'un des champs de données masqués est altéré, dans lequel les champs de données masqués sont altérés en démasquant les champs de données masqués ; et
b. si l'un des champs de données masqués est altéré, générer une alerte indiquant au patient l'altération des champs de données masqués.

8. Système (100) selon la revendication 6, dans lequel, lors du masquage des champs de données dans les informations du patient, le module de masquage (103) est configuré pour :
a. déterminer si l'un des champs de données dans les informations du patient est masqué selon les préférences définies par un fournisseur de services de soins de santé;
b. si l'un des champs de données est masqué, comparer les champs de données masqués avec le ou les champs de données spécifiés dans les préférences du patient ;
c. déterminer les champs de données qui doivent être masqués et les champs de données qui ne doivent pas être masqués sur la base des préférences du patient ;
d. masquer les champs de données qui sont déterminés comme devant être masqués selon les préférences du patient ; et
e. démasquer les champs de données masqués qui ne sont pas déterminés comme devant être masqués selon les préférences du patient.

9. Système (100) selon la revendication 6, comprenant en outre un module de préférence (104) configuré pour paramétrer les préférences du patient en ce qui concerne la confidentialité des informations du patient en tant que préférences par défaut pour masquer les informations du patient.

10. Support de stockage lisible par ordinateur non transitoire ayant des instructions lisibles par machine stockées dans celui-ci, qui, lorsqu'elles sont exécutées par un serveur (101), amènent le serveur (101) à exécuter les étapes du procédé comprenant :
- obtenir un dossier médical associé à un patient à partir d'une base de données de dossiers médicaux (102), dans lequel le dossier médical comprend un identifiant de patient, des informations sur la santé du patient et des données médicales ;
- déterminer les préférences du patient en ce qui concerne la confidentialité des informations du patient dans le dossier médical ;
- masquer un ou plusieurs champs de données dans les informations du patient sur la base des préférences déterminées ; et
- générer le dossier médical contenant les champs de données masqués dans les informations du patient,
dans lequel, lors du masquage des champs de données dans les informations du patient, les instructions amènent le processeur (202) à exécuter les étapes du procédé comprenant :
déterminer un niveau de confidentialité associé à chacun des champs de données des informations du patient sur la base des préférences concernant la confidentialité du patient ; et
masquer le ou les champs de données dans les informations du patient sur la base d'un niveau de confidentialité,
où le masquage comprend cacher ou rendre anonymes des champs de données du dossier médical en supprimant, modifiant ou effaçant les informations contenues dans les champs de données relatifs aux informations du patient,
dans lequel, en déterminant les préférences du patient en ce qui concerne la confidentialité des informations du patient, les instructions amènent le serveur (101) à exécuter les étapes du procédé comprenant :
a. déterminer si des préférences sont spécifiées par le patient en ce qui concerne la confidentialité des informations du patient sur la base de l'identifiant du patient ;
b. si des préférences sont spécifiées, déterminer un ou plusieurs champs de données dans les informations du patient à masquer sur la base des préférences spécifiées par le patient ; et **caractérisé en ce que**
c. si aucune préférence n'est spécifiée, demander au patient de fournir les préférences en ce qui concerne la confidentialité des informations du patient.

11. Support de stockage selon la revendication 10, dans lequel les instructions amènent le serveur (101) à exécuter les étapes du procédé comprenant :
a. déterminer si l'un des champs de données masqués est altéré ; et
b. si l'un des champs de données masqués est altéré, générer une alerte indiquant au patient l'altération des champs de données masqués.

12. Support de stockage selon l'une des revendications précédentes 10 à 11, dans lequel les instructions amènent le serveur (101) à exécuter les étapes du procédé comprenant :
a. déterminer si l'un des champs de données dans les informations du patient est masqué selon les préférences définies par un fournisseur de services de soins de santé;
b. si l'un des champs de données est masqué, comparer les champs de données masqués avec le ou les champs de données spécifiés dans les préférences du patient ;
c. déterminer les champs de données qui doivent être masqués et les champs de données qui ne doivent pas être masqués sur la base des préférences du patient ;
d. masquer les champs de données qui sont déterminés comme devant être masqués selon les préférences du patient ; et
e. démasquer les champs de données masqués qui ne sont pas déterminés comme devant être masqués selon les préférences du patient.
